# EUROPEAN PATENT APPLICATION

(11) **EP 1 020 420 A1**
(43) Date of publication of application: **19.07.2000**
(21) Application number: 99926960.8
(22) Date of filing: 06.07.1999
(51) Int. Cl.: C07C 13/68, C07C 2/50

(54) **1,4-METHANO-1,4,4a,9a-TETRAHYDROFLUORENE COMPOSITION AND PROCESS FOR PRODUCING THE SAME**

(30) Priority: 06.07.1998 JP 20426898; 06.07.1998 JP 20426998
(71) Applicant: Nippon Petrochemicals Company, Limited, Tokyo 100-8530 (JP)
(72) Inventor: AIDA, Fuyuki, Yokohama-shi, Kanagawa 233-0007 (JP); SUZUKI, Takashi, Yokohama-shi, Kanagawa 235-0016 (JP); INOMATA, Yoshihisa, Yokohama-shi, Kanagawa 230-0021 (JP); MATSUMURA, Yasuo, Yokohama-shi, Kanagawa 245-0018 (JP)
(74) Representative: Strehl Schübel-Hopf & Partner
(86) International application number: JP9903651
(87) International publication number: WO0001646

(57) **Abstract**

Cyclopentadiene and/or dicyclopentadiene and indene as much as 2 to 50 times by mol of said cyclopentadiene are reacted under heating, and the obtained reaction product is then refined by fractional distillation to obtain a composition of 1,4-methano-1,4,4a,9a-tetrahydrofluorene, in which the content of two types of cyclopentadiene trimers having specific structures is limited to 100 to 5,000 ppm. Polymer having excellent optical property can be obtained due to the suppressing of gelation in the polymerization when said composition is employed as a raw material for various polymerization.

## Description

### Technical Field

The present invention relates to a 1,4-methano-1,4,4a,9a-tetrahydrofluorene composition in which the content of cyclopentadiene trimer having a specific structure is limited, and a process for producing the same.

### Background Art

In recent years, it has been known that cycloolefin typically exemplified by 1,4-methano-1,4,4a,9a-tetrahydrofluorene is very useful as a raw material for preparing resins having excellent optical property; heat resistance, oil absorption property and the like. These cycloolefins are used for polymerization in the presence of organometallic complex catalyst, wherein the polymerization method is roughly classified into two types. One is ring opening metathesis polymerization and the other is homopolymerization at the olefinic sites of these cycloolefins or copolymerization of lower α-olefins which is carried out by using Ziegler catalyst or metallocene catalyst.

It is proposed to produce 1,4-methano-1,4,4a,9a-tetrahydrofluorene through the addition reaction of indene and cyclopentadiene and to employ the resultant 1,4-methano-1,4,4a,9a-tetrahydrofluorene as a raw material for polymerization.

For example, Japanese Laid-Open Patent Publication No. 5-97719 discloses that resins excellent in optical property can be produced through the copolymerization of 1,4-methano-1,4,4a,9a-tetrahydrofluorene with olefin such as ethylene in the presence of vanadium catalyst or metallocene catalyst. Furthermore, Japanese Laid-Open Patent Publication N0. 7-53680 discloses that resins having excellent optical property can be obtained by ring opening polymerization of 1,4-methano-1,4,4a,9a-tetrahydrofluorene, followed by hydrogenation or cyclization. Generally, in Diels-Alder reaction of indene and cyclopentadiene, cyclopentadiene trimers are formed as by-products in addition to the 1,4-methano-1,4,4a,9a-tetrahydrofluorene. It is difficult to separate cyclopentadiene trimers from 1,4-methano-1,4,4a,9a-tetrahydrofluorene by commercial separation method such as distillation because of the small difference in boiling points between them. Consequently, the above-mentioned trimers are often contained as impurities.

Furthermore, it has been found out that such impurities may cause gelation in the course of the polymerization and they deteriorate the optical property of the obtained polymer.

The object of the invention is thus to provide a 1,4-methano-1,4,4a,9a-tetrahydrofluorene composition which is polymerizable without substantially deteriorating the optical property of the polymers due to the gelation.

### Disclosure of Invention

A first aspect of the present invention relates to 1,4-methano-1,4,4a,9a-tetrahydrofluorene composition represented by the following formula [I] wherein the total content of cyclopentadiene trimers represented by the following formulae [II] and [III] is from 100 to 5,000 ppm.

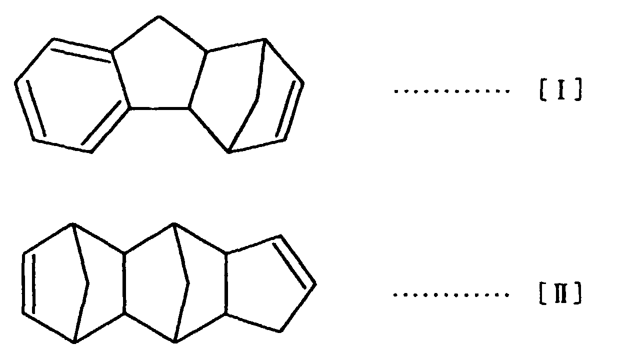

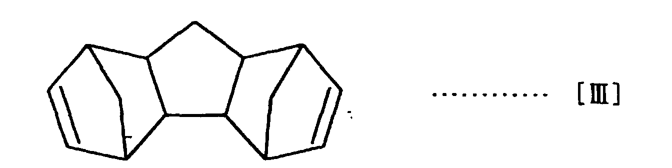

A second aspect of the present invention relates to 1,4-methano-1,4,4a,9a-tetrahydrofluorene composition as mentioned in the first aspect of the invention wherein the content of cyclopentadiene trimer represented by the above formula [III] is from 100 to 3,000 ppm.

A third aspect of the present invention relates to a process for producing a composition of 1,4-methano-1,4,4a,9a-tetrahydrofluorene that is represented by the above formula [I], in which composition the total content of cyclopentadiene trimers represented by the above formulae [II] and [III] is from 100 to 5,000 ppm, and said process comprises the steps of: heating and reacting cyclopentadiene and/or dicyclopentadiene with 2 to 50 times by mole of indene on the basis of the cyclopentadiene; refining by distillation the resultant reaction mixture; and recovering a fraction containing a main component boiling in the range of 250 to 300°C under the normal pressure.

A fourth aspect of the present invention relates to a process for producing the composition of 1,4-methano-1,4,4a,9a-tetrahydrofluorene that is represented by the formula [I], wherein the content of cyclopentadiene trimer represented by the formula [III] is 100 to 3,000 ppm in the cyclopentadiene trimers as mentioned in the third aspect of the invention.

A fifth aspect of the present invention relates to a process for producing the composition of 1,4-methano-1,4,4a,9a-tetrahydrofluorene that is represented by the formula [I], which is characterized in that inert hydrocarbon solvent is used in the process of mixing, heating and reacting cyclopentadiene and/or dicyclopentadiene and indene in the third aspect of the invention.

A sixth aspect of the present invention relates to a process for producing a composition of 1,4-methano-1,4,4a,9a-tetrahydrofluorene that is represented by the formula [I], wherein, in the fifth aspect of the invention, the weight ratio of cyclopentadiene and/or dicyclopentadiene with respect to the total weight of cyclopentadiene and/or dicyclopentadiene, indene and inert hydrocarbon solvent, is in the range of 0.02 to 0.20.

A seventh aspect of the present invention relates to a process for producing a composition of 1,4-methano-1,4,4a,9a-tetrahydrofluorene as represented by the formula [I], wherein, in the fifth aspect of the invention, the reaction temperature is in the range of 100 to 350°C, the reaction pressure is from the normal pressure to 10 MPa and the residential time is from 0.01 to 100 hours.

An eighth aspect of the present invention relates to a process for producing a composition of 1,4-methano-1,4,4a,9a-tetrahydrofluorene as represented by the formula [I], in the fifth aspect of the invention, which process is characterized in that unreacted cyclopentadiene, dicyclopentadiene, indene and inert hydrocarbon solvent are recycled and reused.

The present invention will be described in more detail.

The composition of the 1,4-methano-1,4,4a,9a-tetrahydrofluorene as represented by the formula [I] according to the present invention can be synthesized by subjecting cyclopentadiene and/or dicyclopentadiene and indene to thermal addition reaction such as Diels-Alder reaction.

The reaction mixture to be obtained contains unreacted cyclopentadiene, dicyclopentadiene and indene, and also cyclopentadiene trimers represented by the formulae [II] and [III] as well as the intended product of 1,4-methano-1,4,4a,9a-tetrahydrofluorene. The cyclopentadiene trimers have boiling points close to that of the 1,4-methano-1,4,4a,9a-tetrahydrofluorene, so that they are apt to be involved in the 1,4-methano-1,4,4a,9a-tetrahydrofluorene in the purification process through distillation. The cyclopentadiene trimers comprise diolefin compounds represented by the formula [II], which contains a norbornene-type olefinic structure and cyclopentene-type olefinic structure, and diolefin compounds represented by the formula [III], which contain a pair of norbornene-type olefinic structure. It has been found that these diolefins deteriorate the optical property of the polymers to be obtained not only through ring opening metathesis polymerization but also in homopolymerization of cycloolefins at olefinic sites in the presence of Ziegler catalyst or copolymerization with lower α-olefin.

Accordingly, the total content of the above-mentioned diolefins in the composition of 1,4-methano-1,4,4a,9a-tetrahydrofluorene as represented by the above formula [I] of the present invention, is in the range of 100 to 5,000 ppm, preferably from 100 to 4,000 ppm, and more preferably from 100 to 3,000 ppm. Further, the content of cyclopentadiene trimer as represented by the formula [III] is in the range of 100 to 3,000 ppm, preferably from 100 to 2,500 ppm, and more preferably from 100 to 2,000 ppm.

When the content of cyclopentadiene trimers exceeds the upper limit of these range, the cross-linking reaction takes place in the course of not only ring opening metathesis polymerization but also homopolymerization of cycloolefins at olefinic sites thereof or copolymerization with lower α-olefin. Thus, in the obtained polymers, the polydispersed structure is notable and gelation is liable to occur with high possibility. When the content of cyclopentadiene trimers is reduced to a level less than the lowest limit of the above-mentioned range, it is not economically preferable because it imposes high loads to refining by distillation, which is undesirable because it necessitates the increase in the number of plates or the amount of refluxing of a distillation tower.

When cyclopentadiene is used, it can be obtained by previously subjecting dicyclopentadiene to thermal cracking distillation. It is also possible to feed directly dicyclopentadiene itself and it is converted into cyclopentadiene in the reaction system by thermal cracking. As dicyclopentadiene, commercially available one having purity of desirably not less than 90% can be used. The impurities in dicyclopentadiene are mainly tetrahydromethylindene as well as the addition products of C₅ to C₉ normal or cyclic diolefins with cyclopentadiene. When the content of these impurities is large, the formation of cyclopentadiene trimers and other higher boiling by-products increases. It is thus preferable to employ highly pure starting materials.

Indene which can be used in the present invention, has purity of preferably not less than 30%, more preferably not less than 60%, and still more preferably 90% or more. The content of impurities such as benzonitrile, benzoindene and dialkylbenzene is allowable.

Cyclopentadiene and dicyclopentadiene are introduced into a reaction system using a booster and a pump. It is possible to mix previously with other raw material of indene, or these materials may be fed separately. However, when these raw materials are fed separately, two raw material tanks and two pumps are required, so that it is preferable to mix them in advance.

With regard to the feeding ratio of indene and cyclopentadiene, the molar ratio in terms of indene/cyclopentadiene is from 2 to 50, preferably from 2 to 40, and more preferably from 2 to 20. The amount of dicyclopentadiene is herein calculated by mol in terms of cyclopentadiene. When the amount of indene exceeds the upper limit of the above range, it is not preferable because unreacted indene increases. On the contrary, when the amount of cyclopentadiene or dicyclopentadiene exceeds the lower limit of the above range, the cyclopentadiene trimers represented by the formulae [II] and [III] and other high boiling compounds are formed, and furthermore, the rate of effective utilization of raw materials may be reduced. A solvent can be used in the production of the composition of the present invention. The purpose of use is to reduce the formation of high boiling compounds as by-products with diluting the concentrations of components. Suitable solvents are exemplified by hydrocarbons having 6 to 13 carbon atoms. Among them, the widely used normal hexane, isohexane, normal heptane, isoheptane, normal octane, isooctane, normal nonane, isononane, normal decane, normal undecane, normal dodecane, normal tridecane, beuzene, toluene, xylene, ethylbenzene, trimethylbenzene, diethylbenzene, triethylbenzene, cyclohexane, methylcyclohexane, dimethylcyclohexane, and ethylcyclohexane can be preferably used. These solvents may be used as a mixture of two or more of them.

The solvent to be used preferably has a difference in boiling point of 5°C or more as compared with those of cyclopentadiene or dicyclopentadiene, indene, and 1,4-methano-1,4,4a,9a-tetrahydrofluorene. In the practical use of such a solvent, it is preferable to mix it with indene, or with indene, cyclopentadiene and dicyclopentadiene prior to the feeding into reaction system.

It is preferable that the weight ratio of cyclopentadiene and/or dicyclopentadiene to the total weight of cyclopentadiene and/or dicyclopentadiene, indene and inert hydrocarbon solvent is from 0.02 to 0.20. When this ratio is less than 0.02, the productivity of intended product is low, meanwhile if it exceeds 0.2, the formation of high boiling compounds increases. So that, either case is not desirable.

The synthesis of the 1,4-methano-1,4,4a,9a-tetrahydrofluorene is carried out using these compounds as raw materials, and if necessary with a solvent. The process for production is not particularly restricted, and any suitable method can be employed.

The operation of the reaction according to the present invention can be achieved in any of batch system and flow-type continuous system. From the industrial viewpoint, it is preferable to select the flow-type continuous operation.

Concerning the reactor, any of perfect mixing type and piston flow type ones can be used. Examples of commercially available reactor include "Static Mixer" manufactured by Noritake Co., Ltd.; "Sulzer Mixer" manufactured by Sumitomo Heavy Industries Ltd.; and "Square Mixer" manufactured by Sakura Seisakusho Ltd. The reaction can be carried out either in one stage system or multi-stage system having two or more stages. It is also possible to use the reactors of perfect mixing type and piston flow type by connecting them in parallel or in series.

Reaction temperature is in the range of 100 to 350°C. Particularly, when dicyclopentadiene is used as a starting material, reaction temperature is preferably 100°C or above, and more preferably 150°C or above. When reaction temperature is higher than 350°C, formation of high boiling compounds represented by the formulae [II] and [III] will increase in any case. When cyclopentadiene is used as a starting material, the reaction temperature of 100 to 350°C is preferable.

With regard to the reaction conditions to be employed in the case of a batch reactor, residential time is usually from 0.01 to 100 hr, preferably from 0.05 to 5 hr, and more preferably from 0.1 to 3 hr, while the reaction pressure is in the range of atmospheric pressure to 10,000 kPa.

With regard to the reaction conditions to be employed in the case of a continuous reactor, space velocity is usually from 0.01 to 100 h⁻¹, preferably from 0.05 to 5 h⁻¹, and more preferably from 0.1 to 10 h⁻¹. When space velocity exceeds 100 h⁻¹, it is not proper because of increasing of unreacted materials. Meanwhile, the reaction pressure to be employed is in the range of atmospheric pressure to 10,000 kPa.

The reaction mixture taken out from the reactor is introduced into purification process. Although there are several purification methods such as chromatographic separation, crystallization, distillation and so forth, preferred one is distillation. It can be operated batch-wise or continuously. The continuous distillation is, however, preferable commercially.

In the case of the batchwise process, residual cyclopentadiene, solvent (when used), dicyclopentadiene and indene can be separated one by one. Otherwise, three or four components are separated simultaneously. In order to enhance the separation efficiency, the distillation tower can be packed with various packing materials or refluxing can be employed. The theoretical plate number of each distillation tower is from 1 to 100, preferably from 2 to 50, and more preferably from 3 to 30. The reflux ratio is determined according to the condition of separation in a distillation tower and it may be suitable in the range of 1 to 50.

In the batch distillation process, the residual cyclopentadiene is separated in the first place from the reaction mixture that is taken out from the reactor. As the distillation conditions, temperature can be arbitrarily selected from the range of 0 to 200°C and the pressure, from 0.01 to 1,000 kPa, respectively.

In the next step, solvent (when used), residual dicyclopentadiene and indene are separated. As distillation conditions, temperature of 30 to 150°C and the pressure of 10 kPa or lower are suitable.

Furthermore, concerning distillation conditions for separating the intended product of 1,4-methano-1,4,4a,9a-tetrahydrofluorene, temperature is in the range of 50 to 200°C and the pressure is 1 kPa or lower.

High boiling compounds are obtained from the bottom residue of the distillation tower.

Then, as the method for simultaneously separating two to three components, the residual cyclopentadiene, dicyclopentadiene and indene are separated at the same time. As distillation conditions, temperature of 30 to 150°C and the pressure of 10 kPa are suitable. As distillation conditions for separating the 1,4-methano-1,4,4a,9a-tetrahydrofluorene, temperature of 50 to 200°C and the pressure 1 kPa or lower.

In the case of continuous distillation process, it is preferable to employ at least two distillation towers. When two distillation towers are used, in the first tower, cyclopentadiene, solvent (when used), residual dicyclopentadiene, and indene are recovered from the top of the tower and the mixture at the bottom is introduced into the second tower. The intended product of 1,4-methano-1,4,4a,9a-tetrahydrofluorene is then obtained from the top of the second tower, while high boiling compounds are obtained from the bottom of the second tower.

In this case, as distillation conditions in the first tower, temperature of 30 to 150°C and pressure of 10 kPa or lower are suitable when dicyclopentadiene and indene still remain. When they do not remain, the pressure of 1 to 100 kPa and the temperature of 20 to 140°C are suitable.

As distillation conditions in the second tower, temperature of 50 to 200°C and pressure of not higher than 1 kPa are employed. When cyclopentadiene and indene are absent, cyclopentadiene and solvent (when used) are recovered under conditions of pressure of 1 to 100 kPa and temperature of 20 to 140°C. When dicyclopentadiene contains impurities, it is preferable to carry out an additional operation to remove them under conditions of temperature of 30 to 150°C and pressure of not higher than 10 kPa. It is, thereby, possible to maintain high purity of 1,4-methano-1,4,4a,9a-tetrahydrofluorene.

When three distillation towers are used, the cyclopentadiene is mainly separated from the top of first tower. As distillation conditions, pressure and temperature can be arbitrarily selected from the ranges of 0.1 to 1MPa and 0 to 100°C, respectively.

The mixture obtained from the bottom of the tower is introduced into the second tower. Solvent (when used) and residual dicyclopentadiene and indene are simultaneously recovered from the top of the second tower. When dicyclopentadiene and indene still remain, temperature of 30 to 150°C and pressure of not higher than 10 kPa are suitable. While, if they do not remain, pressure of 1 to 100 kPa and temperature of 20 to 140°C are suitable.

The mixture obtained from the bottom of the second tower is introduced into the third tower. 1,4-Methano-1,4,4a,9a-tetrahydrofluorene is separated and purified at the top of the third tower under conditions of temperature of 50 to 200°C and pressure of 1 kPa or lower. When no dicyclopentadiene remains, solvent (when used) is recovered under distillation conditions of pressure of 1 to 100 kPa and temperature of 20 to 140°C. When dicyclopentadiene contains impurities, however, it is preferable to perform an additional removal operation under conditions of temperature of 30 to 150°C and pressure of not higher than 10 kPa. Through this operation, it is possible to maintain purity of 1,4-methano-1,4,4a,9a-tetrahydrofluorene at a high level.

When four distillation towers are used, cyclopentadiene is separated from the top of the first tower. Distillation conditions can be arbitrarily selected from the range of pressure of 0.1 to 1 MPa and temperature of 0 to 100°C.

The mixture obtained from the bottom of the tower is introduced into the second tower, and solvent (when used) is recovered from the top of the tower. Distillation conditions employed are pressure of 1 to 100 kPa and temperature of 20 to 140°C.

The mixture obtained from the bottom of this tower is introduced into the third tower, and the mixture composed of main components of dicyclopentadiene and indene is separated from the top of the tower. As distillation conditions in the third tower, temperature of 30 to 150°C and pressure of 10 kPa or lower are suitable.

The mixture obtained from the bottom of the third tower is introduced into the fourth tower, and 1,4-methano-1,4,4a,9a-tetrahydrofluorene is separated under conditions of temperature of 50 to 200°C and pressure of not higher than 1 kPa and high boiling compounds as the reaction by-products are obtained from the bottom of the tower.

In another method of employing four distillation towers, cyclopentadiene is separated from the top of the first tower As distillation conditions, pressure in the range of 0.1 to 1 MPa and temperature in the range of 0 to 100°C can be arbitrarily selected.

The mixture obtained from the bottom of this tower is introduced into the second tower, and solvent (when used), dicyclopentadiene and indene are simultaneously recovered from the top of the tower. Distillation conditions are such that temperature of 30 to 150°C and pressure of 10 kPa or lower.

The mixture obtained from the top of this tower is introduced into the third tower, and the solvent (when used) is separated from the top of the tower, and dicyclopentadiene and indene are separated from the bottom of the tower. As distillation conditions for the third tower, pressure of 1 to 100 kPa and temperature of 20 to 140°C are suitable.

The mixture obtained from the bottom of the second tower is introduced into the fourth tower. 1,4-Methano-1,4,4a,9a-tetrahydrofluorene is separated from the top of the tower under conditions of temperature of 50 to 200°C and pressure of not higher than 1 kPa. High boiling compounds as the reaction by-products are obtained from the bottom of the tower.

In either batch or continuous operation, it is important to conduct distillation while maintaining the maximum temperature of 200°C or below because the 1,4-methano-1,4,4a,9a-tetrahydrofluorenes are liable to be decomposed at higher temperatures.

Furthermore, when solvent is used, distillation conditions are varied because boiling points of solvents differ depending on the kinds of them.

In order to enhance separation efficiency, it is possible to fill each distillation tower with various kinds of packing materials or to carry out refluxing. The theoretical number of plates of each distillation tower is from 1 to 100, preferably from 2 to 50, and more preferably from 3 to 30. The suitable ratio of refluxing is determined according to condition of separation in each distillation tower and a ratio in the range of 1 to 50 is suitable.

It is preferable to reuse the unreacted cyclopentadiene, dicyclopentadiene, and indene that are separated in the distillation process and solvent which is also separated.

1,4-Methano-1,4,4a,9a-tetrahydrofluorene produced by the foregoing method, in which the content of cyclopentadiene trimers as represented by the formulae [II] and [III] is reduced, is an isomer mixture of endo isomer and exo isomer with the endo /exo molar ratio of 80/20 to 60/40.

Furthermore, in order to carry out the reaction of the present invention, it is possible to add optionally antioxidants, polymerization inhibitors or the like to reactants. For example, phenolic compounds such as hydroquinone, 2,6-di-tert-butylphenol, 2,6-di-tert-butyl-p-cresol and 4-methoxyphenol, hydroxylamine compounds such as N,N-dimethylhydroxylamine and N,N-diethylhydroxylamine can be optionally added. The addition quantity of them is in the range of 10 to 10,000 ppm, preferably from 50 to 5,000 ppm with respect to the total amount of the reactants fed into a reactor. They can also be added optionally to the 1,4-methano-1,4,4a,9a-tetrahydrofluorene composition that is an intended product of the present invention.

The 1,4-methano-1,4,4a,9a-tetrahydrofluorene composition of the present invention produced as described above can be used in metathesis polymerization or in the polymerization in the presence of a Ziegler catalyst according to conventionally known methods.

Metathesis polymerization can be carried out according to the method disclosed, for example, in Japanese Laid-Open Patent Publication Nos. 9-183832 and 8-151435, U. S. Patent Nos. 5,462,995 and 5,580,934, Japanese Laid-Open Patent Publication Nos. 1-168725, 1-168724 and 60-026024. More particularly, the methods are done using catalysts comprising (a) catalyst components of transition metal compounds and (b) catalyst promoters of organometallic compounds.

The catalyst components of transition metal compounds (a) are the compounds of transition metals belonging to Groups IVB, VB, VIB, VIIB or VIII of the periodic table, and more particularly, there are exemplified by halides, oxyhalides, alkoxyhalides, alkoxides, carboxylates, (oxy)acetylacetonates, carbonyl complexes, acetonitrile complexes, hydride complexes, derivatives of them, and complexes of the phosphine compounds of them.

The catalyst promoters of organometallic compounds (b) are compounds of metals belonging to Groups IA, IIA, IIB IIIA, and IVA of the periodic table, each having at least one metal-carbon bond or metal-hydrogen bond such as the organic compounds of Al, Sn, Li, Na, Mg, Zn, Cd and B.

Furthermore, in addition to both the above components (a) and (b), in order to enhance the activity of metathesis polymerization, it is possible to add aliphatic tertiary amine, aromatic tertiary amine, molecular oxygen, alcohol, ethers, peroxide, carboxylic acid, acid anhydride, acid chloride, ester, ketone, nitrogen-containing compounds, sulfur-containing compounds, halogen-containing compounds, molecular iodine, or Lewis acid.

Meanwhile, homopolymerization of at the olefinic site of tetracyclodecene or copolymerization with lower α-olefin can be carried out using a Ziegler catalyst containing metallocene catalyst.

Polymerization by using metallocene catalyst can be carried out according to the methods disclosed, for example, in U.S. Patent No. 5,087,677, Japanese Laid-Open Patent Publication No. 2-173112, U.S. Patent No. 5,344,900, European Patent No. 0283164 A, and Japanese Laid-Open Patent Publication No. 61-221206. More particularly, there are methods using a catalyst consisting of (c) a component of the compound of transition metal belonging to Group IVB, VB or VIB of the periodic table and (d) a component of organoaluminum oxy compound.

The component of transition metal compound of (c) is a compound of a transition metal belonging to Groups IVB, VB or VIB of the periodic table. This type of compounds have at least two cycloalkadienyl groups or their substituted compounds, or these cycloalkadienyl groups or their substituted compounds are bonded through hydrocarbon groups, silylene groups or substituted sylilene groups, so called multidentate ligands.

(d) The component of organoaluminum oxy compound is a compound represented by the following formulae [IV] and [V]. In these formulae, R denotes hydrocarbon, more particularly, methyl, ethyl, propyl, and butyl, and m is an integer of 2 or more.

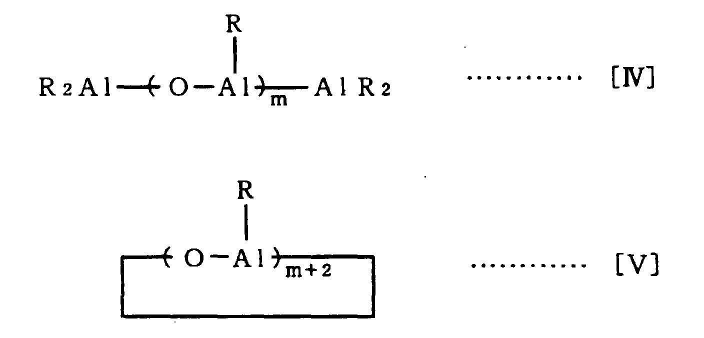

As Ziegler catalyst, compound of titanium or vanadium is employed. For example, as disclosed in Japanese Laid-Open Patent Publication No. 9-176396 and European Patent No. 0203799 A, a copolymer of cycloolefin with α-olefin such as ethylene can be synthesized using a catalyst formed of vanadium compound and an organoaluminum compound that are soluble in a hydrocarbon solvent.

The composition of the present invention can be polymerized by means of any of the above-described polymerization methods with less gelation and with less possibility of deterioration in optical property of the resultant polymers.

In ring opening polymerization, the obtained polymer often contains unsaturated bonds, and as a result, heat resistance and light resistance of the polymer are poor. In such a case, in order to improve these properties, a part of or all of unsaturated bonds can be hydrogenated. Hydrogenation is carried out using a nickel or a precious metal of palladium or platinum that is dispersed and supported on inorganic carrier. Homogeneous hydrogenation catalysts can also be used. As hydrogenation conditions, pressure of 0.1 to 20 MPa and temperature of 0 to 200°C are taken.

### Best Method to Carry Out the Embodiment of the Invention

The present invention will be described in more detail with reference to the following examples. In the description, "%" indicates "mass %" unless otherwise specified.

### 〈Example 1〉

Indene (94.2% purity), dicyclopentadiene (94.7% purity) and methylcyclohexane were mixed in a weight ratio of 44/6/50 and the mixture was continuously introduced into a 50 ml autoclave and the reaction product was taken out continuously while maintaining a pressure at 5,000 kPa. The reaction was continued for 5 hours at 230°C under space velocity of 4 h⁻¹. The reaction product was purified under the conditions of reflux ratio of 10 and at 0.07 kPa, as a result, 72g of a fraction in the range of 88 to 92°C was obtained. The purity of 1,4-methano-1,4,4a,9a-tetrahydrofluorene was 99.6% as determined by gas chromatographic analysis. The above fraction was found to involve 2,600 ppm of the compound represented by the formula [II] and 1,200 ppm of the compound represented by the formula [III].

Into a 2,000 ml round bottle flask equipped with stirring blades was introduced 1,000 ml of toluene that was dried under the nitrogen atmosphere. Furthermore, into this flask were added 1 mmol of dichloroethoxyoxo vanadium and 10g of 1,4-methano-1,4,4a,9a-tetrahydrofluorene, which tetrahydrofluorene was previously synthesized and contained 2,600 ppm of a compound represented by the formula [II] and 1,200 ppm of a compound represented by the formula [III]. This solution was subjected to bubbling with stirring and supplying gas mixture of ethylene and nitrogen (molar ratio of ethylene/nitrogen: 1:5) at a flow rate of 200 l/h. Furthermore, polymerization was started by adding drop wise 10 mmol of ethylaluminum sesquichloride, and continued for 20 min. at 5°C.

The polymerization was terminated by adding 30 ml of methanol and copolymer was precipitated by pouring the reaction mixture into 2,000 ml of methanol. The copolymer after being dried under reduced pressure was molded into a pressed sheet of 1 mm thickness at 240°C. The thus obtained sheet was transparent and no gelation was observed.

### 〈Comparative Example 1〉

The same test as in Example 1 was carried out except that a mixture of indene, dicyclopentadiene and n-hexane in a weight ratio of 32/18/50 was used as starting materials. The reaction product was subjected to fractionation under the conditions of reflux ratio of 10 and at 0.07 kPa. As a result, 118g of a fraction in the range of 88 to 92°C was obtained. The purity of 1,4-methano-1,4,4a,9a-tetrahydrofluorene was 96.2% as determined by gas chromatographic analysis. This fraction was found to involve 27,100 ppm of a compound represented by the formula [II] and 10,100 ppm of a compound represented by the formula [III].

The copolymerization with ethylene was carried out similarly as in Example 1 except that 1,4-methano-1,4,4a,9a-tetrahydrofluorene composition as obtained in the above process was used and pressed sheet was formed. It was confirmed that gel existed in the sheet. A portion of the gel was then cut off and heated up to 300°C on a hot plate but it did not melt.

### 〈Example 2〉

20g of 1,4-methano-1,4,4a,9a-tetrahydrofluorene composition, 2.5 g of norbornene, 2.5 g of dicyclopentadiene and 200 ml of toluene were introduced into a thoroughly dried 500 ml autoclave. The used tetrahydrofluorene composition was the one which was synthesized in Example 1 containing 2,600 ppm of the compound represented by the formula [II] and 1,200 ppm of a compound represented by the formula [III]. After that, 3 mmol of triethyl aluminum was added to it and then 0.7 mmol of titanium tetrachloride and 7 mmol of triethylamine were added to this and ring opening polymerization was carried out at 30°C for 6 hours. Polymer was precipitated by pouring the reaction product into a mixture of acetone and isopropyl alcohol and the obtained polymer was dissolved again in toluene. Operation of precipitation was repeated twice and the precipitate was dried under reduced pressure to obtain 21g of polymer.

Into a 200ml autoclave were introduced 0.4g of 0.5% palladium carbon and 100 ml of toluene and 4g of polymer obtained through the ring opening polymerization was further added. Hydrogenation was carried out at 20°C for 1 hour with 5 MPa of the pressure of hydrogen. After the reaction, the catalyst was removed by filtration and re-precipitation was done using methanol.

The obtained copolymer was dried under reduced pressure and formed into pressed sheet of 1 mm in thickness at 350°C. The sheet was homogeneous and transparent and any gelation was not observed.

### 〈Comparative example 2〉

Polymerization and hydrogenation were carried out in the like manner as in Example 2 except that 1,4-methano-1,4,4a,9a-tetrahydrofluorene composition as used in Comparative Example 1, which contained 27,100 ppm of a compound represented by the formula [II] and 10,100 ppm of a compound represented by the formula [III], was used to obtain a polymer. Gelation was observed in the pressed sheet that was formed with the obtained polymer.

### Industrial Applicability

When the 1,4-methano-1,4,4a,9a-tetrahydrofluorene composition of the present invention is used as a raw material for polymerization, the formation of gel can be substantially avoided because the content of cyclopentadiene trimers is small. Therefore, polymer having excellent optical property can be obtained.

## Claims

1. A composition of 1,4-methano-1,4,4a,9a-tetrahydrofluorene represented by the following formula [I], wherein the content of cyclopentadiene trimers represented by the following formulae [II] and [III] is in the range of 100 to 5,000 ppm.

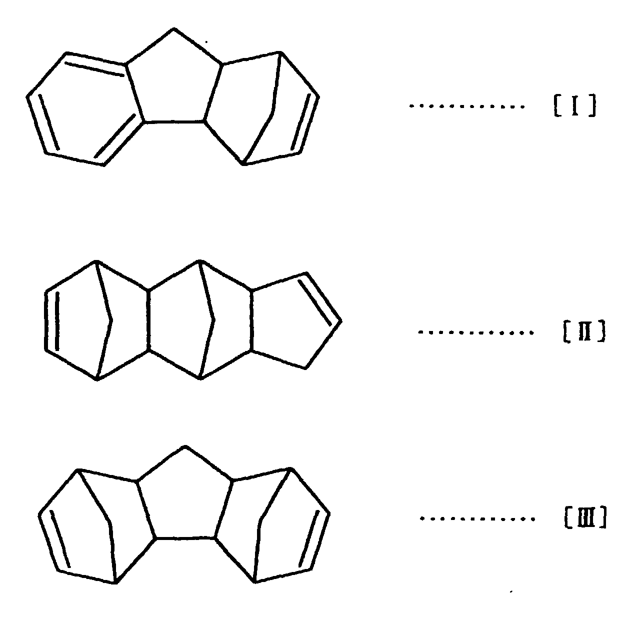

2. A composition of 1,4-methano-1,4,4a,9a-tetrahydrofluorene in Claim 1, wherein the content of cyclopentadiene trimer represented by the above formula [III] is in the range of 100 to 3,000 ppm.

3. A process for producing a composition of 1,4-methano-1,4,4a,9a-tetrahydrofluorene represented by the following formula [I], wherein the content of cyclopentadiene trimers represented by the following formulae [II] and [III] is in the range of 100 to 5,000 ppm, which process comprises the steps of subjecting cyclopentadiene and/or dicyclopentadiene and indene as much as 2 to 50 times by mol of said cyclopentadiene to reaction under heated condition, fractionating the obtained reaction mixture, and recovering a fraction mainly containing the components in the boiling range of 250 to 300°C under atmospheric pressure.

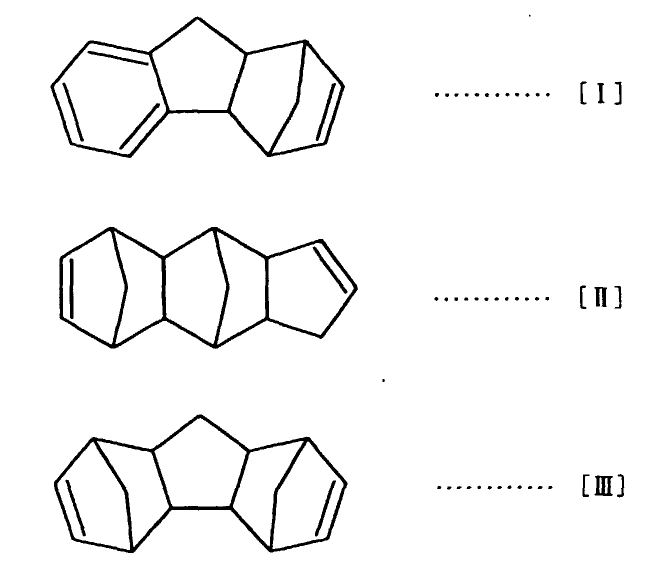

4. A process for producing a composition of 1,4-methano-1,4,4a,9a-tetrahydrofluorene represented by the above formula [I] in Claim 3, wherein the content of cyclopentadiene trimer represented by the above formula [III] is in the range of 100 to 3,000 ppm.

5. A process in Claim 3, which is characterized in that the processes of mixing and reacting with heating of cyclopentadiene and/or dicyclopentadiene and indene are carried out using an inert hydrocarbon solvent.

6. A process in Claim 5, wherein the weight ratio of cyclopentadiene and/or dicyclopentadiene with respect to the total weight of cyclopentadiene and/or dicyclopentadiene, indene and inert hydrocarbon solvent is in the range of 0.02 to 0.20.

7. A process in Claim 5, wherein temperature is in the range of 100 to 350°C, pressure is in the range of the normal pressure to 10 MPa and residential time is in the range of 0.01 to 100 hours in said reaction.

8. A process in Claim 5, which is characterized in that the unreacted cyclopentadiene or dicyclopentadiene, indene and inert hydrocarbon solvent are recycled and reused.
